# EUROPEAN PATENT SPECIFICATION

(11) **EP 4 215 511 B1**
(45) Date of publication and mention of the grant of the patent: **04.03.2026**
(21) Application number: 23152815.9
(22) Date of filing: 23.01.2023
(51) Int. Cl.: C05F 17/20, C05F 17/50, C05F 17/60, C05F 5/00

(54) **PROCESS TO BACTERIALLY DECOMPOSE ORGANIC WASTE MATERIAL**
VERFAHREN ZUR BAKTERIELLEN ZERSETZUNG VON ORGANISCHEM ABFALL
PROCÉDÉ DE DÉCOMPOSITION BACTÉRIENNE DE DÉCHETS ORGANIQUES

(30) Priority: 25.01.2022 NL 2030681
(43) Date of publication of application: 26.07.2023
(73) Proprietor: Eco-Habitat B.V., 1852 AE Heiloo (NL)
(72) Inventor: WIELINGA, Eduard Johannes Christoffel, 1871 AH Schoorl (NL); KREHER, Servan, 1852 AE Heiloo (NL)
(74) Representative: Cramwinckel, Michiel

(56) References cited:
- EP-A1- 3 581 551
- WO-A1-2013/086561
- WO-A1-2014/143359
- WO-A1-2016/064358
- CN-A- 107 986 830
- JP-A- 2005 349 258
- US-A1- 2014 260 464

## Description

The invention is directed to a process to bacterially decompose organic waste material comprising biodegradable plastics to a digested material in a digesting tank in a semi-batch process of between 10 and 30 hours wherein the organic waste material is continuously mixed with aerobic bacteria at a temperature in between 50 C and 70 °C.

Such a process is described in EP3581551. This publication describes a two step process wherein in a first digesting tank 5000 kg biological waste is stirred in the presence of aerobic bacteria for 24 hours at a temperature of 55 C. 80 wt% of the content of this digesting tank was subsequently stirred in a second digesting tank in the presence of the same aerobic bacteria at a temperature of between 45 and 50 C for another 24 hours. The obtained digested material is subsequently dried using a belt dryer combined with heated air. A powdery product is obtained having properties which make it suitable for use as compost for plants.

A disadvantage of the prior art process is the complexity of the process. The two step batch process requires handling of the intermediate product between the two digesting tanks. Further it requires two times 24 hours to obtain the end product. Further the drying steps complicates the process further. WO2013/086561A discloses a process for aerobic digestion of animal effluent with microbes at a temperature of 60 °C under aeration of 2.5 m³ per kg solid material per hour.

The object is to provide a process which is simpler and more efficient.

This object is achieved by the following process. Process to bacterially decompose organic waste material to a digested material in a digesting tank by mixing the organic waste material with aerobic bacteria at a temperature of between 40 C and 70 °C while supplying between 0.5 and 10 m³ of air per hour per kg of the total of organic waste material and digested material and discharging from the digesting tank a humid air stream having at least 60% relative humidity and wherein the organic waste further comprises one or more of the following biodegradable plastics selected from polybutyrate adipate terephthalate (PBAT), polylactic acid (PLA) and/or polyhydroxyalkanates (PHA) and wherein the biodegradable plastic content is in the range of between 1 and 30 %w/w based on 100% dry matter of the organic waste material.

Applicants found that by supplying a large volume of air to the digesting tank a more efficient process is obtained which can convert the organic waste material in one digesting tank in a more efficient manner and no dryer is required.

In the context of the present invention, composting or digesting refers to the degradation of organic waste material into organic matter (compost) by aerobic bacteria in the presence of oxygen. The compost or digestive as obtained in the process is an amorphous humus-like material.

The process is preferably performed as a semi-batch process wherein within a period organic waste material is decomposed to digested material in a digesting tank reactor. The digested material is in whole or partly discharged at the end of the semi-batch process. The process is semi-batch as the air is continuously supplied to the digesting tank and humid air is continuously discharged from the digesting tank. The amount of air flow is suitably made dependent on the air humidity. The semi-batch process may be performed in a relatively short period to a longer period where organic waste material is intermittently added as will be illustrated below.

The organic waste material may be cellulosic, plant and/or animal waste material. The cellulosic, plant and/or animal waste material may suitably be one or more of food substances, biological substances, vegetable waste, animal by-products, such as meat and/or fish waste and/or cellulose rich materials, such as paper, cardboard and sugar cane-based products. The meat waste and/or fish waste is especially an animal by-product category 3 waste. Animal by-products category 3 waste is defined in https://www.gov.uk/guidance/animal-by-product-categories-site-approval-hygiene-and-disposal. Such waste materials may be waste from industry such as restaurant industry, food processing industry; waste from the hospitality industry; waste from the paper industry; waste from recycling facilities; waste from the food or feed industry; waste from the medicinal industry; waste from agriculture or farming related sectors; waste from processing of sugar or starch rich products; contaminated or in other ways spoiled agriculture products such as grain, potatoes and beets not exploitable for food or feed purposes. Waste may also comprise metal or glass pieces.

The organic waste material may be bread and/or dough. The dough may be dough suited to make bread. The bread and dough are preferably waste products such as left overs in retail. It has been found that in the process of this invention the bread and/or dough can be converted into a product which is suited to be used as cattle feed and more advantageously as a fermented bakery product to be used as a substitute for flour to make new bread. It has been found that bread and/or dough can be bacterially decomposed in between 8 and 24 hours.

The plastic in the waste is a biodegradable plastic, namely polybutyrate adipate terephthalate (PBAT), polylactic acid (PLA) or polyhydroxyalkanates (PHA) or their mixtures. Other biodegradable plastic or plastics may also be present. It has been found that at least the listed biodegradable plastics and optionally all cellulose- and lignin-based disposables can be converted in the process of this invention, especially in the presence of some specific aerobic bacteria as described below.

The plastic may be 100% of one type of plastic, may be mixed with other plastics, preferably biodegradable plastic, or may be mixed with other material, for example with plant based material. In one embodiment, the plastic in the waste is used as a lamination for plant fibers, such as sugar cane fibers or bamboo fibers. There is no need to first separate plant fibers and plastic lamination for digestion according to the method of the invention. The plastic may be sturdy, hard, moulded, for example cutlery, trays, cups, tableware; or soft and thin, for example a film or a soft bag.

Polylactic acid (PLA) refers to a thermoplastic polyester with backbone formula (C₃H₄O₂)ₙ, which may be obtained by polycondensation of lactic acid C(CH₃)(OH)HCOOH or through polymerisation of lactide molecules. The lactic acid monomers may be obtained from renewable sources, such as corn starch, cassava starch or sugar cane, by microbial fermentation. Polylactic acid (PLA) may refer to any form of polylactic acid, such as poly(L-)lactic acid (PLLA), poly (D-)lactic acid (PDLA) or poly (D, L-)lactic acid (PDLLA). The polylactic acid may be in crystallised form. Polylactic acid (PLA) is marketed as biodegradable and compostable. In ambient conditions, at home, degrading or composting PLA will takes months or years.

Polybutylene adipate terephthalate (PBAT) is a biodegradable random copolymer, specifically a copolyester of adipic acid, 1,4-butanediol and terephthalic acid.

Polyhydroxyalkanoates (PHA) are polyesters produced in nature by numerous microorganisms, including through bacterial fermentation of sugars or lipids.

In contrast to existing methods for biodegradable plastic degradation, the method according to the invention allows for fast degradation of plastic-containing waste into organic matter and makes the on-site degradation of plastic-containing waste possible.

Examples of organic waste material also comprising plastic may be hospital waste or waste from care institutions, such as waste containing parts or the whole of bandages, clothes, cups, diapers, dressings, filaments, matrices, pins, plates, rods, scaffolds, screws, syringes, threads or tubing of biodegradable plastic.

The organic waste material also comprising the biodegradable plastic is preferably plastic-containing food waste, such as raw or cooked food, food left overs or food scraps, past due date products from supermarkets, such as drinks, vegetables, meat and bones, mixed with plastic packaging, containers or cutlery, preferably mixed with biodegradable plastic packaging, containers or cutlery. Such plastic-containing food waste may be from food services, restaurants, fast food restaurants, hotels, catering services, hospitals, nursing homes, supermarkets, schools, governmental facilities, events, dance festivals or universities.

The plastic in the organic waste material is preferably reduced in size to facilitate digestion, for example by cutting or shredding. A suitable size for the plastic material in the waste is about 1-1.5 square cm. For film like plastic material this accounts to a surface area of between 2 and 3 square cm. Smaller pieces will also yield good results. Larger piece may require longer for complete digestion.

If the waste also comprises metal or glass pieces, these pieces will remain substantially unaffected after the degradation according to the method of the invention, but may be removed, for example by sieving out.

The biodegradable plastic content of the organic waste material is in the range of between 1 and 30 wt. %, preferably between 10 and 30 wt. % or between 20 and 30 wt.% based on 100% dry matter.

The pH of the starting organic waste material is preferably between pH 4.5 and pH 6.

On-site refers to the possibility to process the organic waste material at or near the site where the organic waste material is produced. There is no need to transport the organic waste material for processing in any kind of industrial facility. This saves costs and provides for a reduction in the carbon foot print. The volume reduction of the organic waste material which is achieved with the process according to the invention adds to this advantage. Volume reduction of the organic waste material is preferably at least 70%, at least 80% or at least 85% of the starting volume.

A preferred application of the digested material is as feedstock for an anaerobic digestion to biogas comprising methane and carbon dioxide. It has been found that the digested material may comprise of more than 70 wt% of volatiles and/or has a methane potential of more than 350 litre methane per kg of digested material. Such an anaerobic digestion may be performed using the digested material alone or in combination with manure, for example from livestock. Preferably such an anaerobic digestion is performed on a relatively large scale. By preparing digestive material according to the process of this invention, at or near numerous sites where the organic waste material is produced and one central anaerobic digestion an optimal process is obtained. Instead of using the organic waste material directly as feed in the anaerobic digester the more energy dense digestive material is used. This significantly saves transport costs because of the afore mentioned volume reduction and the anaerobic digester is fed with a feedstock having a more constant quality. A next advantage is that the digestive material is more keepable than the organic waste material. This allows one to use the digestive material more optimal in the anaerobic digestion.

The invention is thus also related to a process to prepare a biogas comprising methane and carbon dioxide from organic waste material by performing a process to bacterially decompose the organic waste material with aerobic bacteria to a digested material, preferably according to this invention, at more than one location, transporting the digestive material to a central location and performing at the central location an anaerobic digestion process to prepare biogas comprising methane and carbon dioxide. Transporting may be along a distance of at least 10 km and suitably less than 100 km. The organic waste material may be as described above. Preferably the organic waste material comprises vegetable waste, meat waste and/or fish waste and/or cellulose rich materials. The meat waste and/or fish waste is also referred to as such as animal by-products category 3 waste.

Another preferred application of the digested material is as part of livestock feed. Especially when the digestive material is prepared from bread and/or dough a digestive material can be prepared which is suitable as a livestock feed component and which complies with ID number 00812-EN of the Feed Materials Register according to EC Regulation No. 767/2009. The invention is especially directed to a process to prepare a livestock feed composition by performing a process to bacterially decompose bread and/or dough, preferably according to this invention, with aerobic bacteria to a digested material, at more than one location, transporting the digestive material to a central location and combining the digestive material with other livestock feed ingredients to prepare the livestock feed composition. Transporting may be along a distance of at least 10 km and suitably less than 100 km. Such a process allows numerous local bakeries and/or retail to process their non-sold bread and/or non-used dough locally to such a digested material, store said material until it is collected and further processed in the central location. Such a process is efficient because the bread and/or dough is converted to a digested material which occupies a substantial lower volume and is keepable. In this way the digested material can be stored until enough digested material is available to be collected and transported to the central location for preparing the livestock feed composition.

The process is performed in the presence of aerobic bacteria. The aerobic bacteria is preferably a bacteria belonging to the genus *Geobacillus.* It has been found that the use of this aerobic bacteria in the process of this invention an even more efficient process is obtained which is also able to convert the above described plastics. This genus includes Gram-positive spore-forming bacteria, some of which were formerly classified within the genus *Bacillus* group 5 and referred to as *Bacilli.* Suitable *Geobacilli* for use in the method according to the invention include *Geobacillus caldoxylosilyticus, Geobacillus kaustophilus, Geobacillus stearothermophilus, Geobacillus subterranens, Geobacillus thermocatenulatus, Geobacillus thermodenitrificans, Geobacillus thermoglucosidans, Geobacillus thermoleovorans* or *Geobacillus uralicus.* Preferred species are thermophilic. In one embodiment, *Geobacillus thermoleovorans* is used, more preferably *Geobacillus thermoleovorans* (Zarilla and Perry 1988; DSM 5366, ATCC 43513). In a preferred embodiment, species of *Geobacillus thermoleovorans* make up at least 80%, at least 85%, at least 90%, at least 95% or at least 99% of the number of bacteria in the composition. In another preferred embodiment, species of *Geobacillus thermoleovorans* make up 100% of the bacteria in the composition.

An even more preferred aerobic bacteria is the *Bacillus* bacteria, such as the *Bacillus Licheniformis.* The *Bacillus Licheniformis* may be present in combination with other aerobic bacteria as for example the bacteria belonging to the genus *Geobacillus* or alone. A preferred *Bacillus Licheniformis* is the *Bacillus Licheniformis* strain BLH20, deposited by the company HTS BIO SA, Parc d'activite de Gemenos, 180-210 avenue de la Roque Forcade, 13420 Gemenos, France, at CNCM (Collection Nationale de Cultures de Microorganismes), Institut Pasteur, 25 rue du Docteur Roux, 75724 Paris Cedex 15, under the Budapest Treaty on 1 April 2021 under registration number CNCM I-5667. It is found that this strain is even more efficient in its ability to quickly decompose plastics and cellulosic, plant and/or animal waste. Mutantes and derived strains of the *Bacillus Licheniformis* and especially of the deposited strain are also suitable for this invention. CNCM I-5667 is a thermophilic Bacteria isolated from a compost obtained during the composting at 60°C of organic waste and PLA (polylactic acid) bioplastic waste in a composting machine. Identification of the genus and bacterial species of CNCM I-5667 was validated by analysis of the 16S rRNA, gyrB and dnaK phylogenetic markers sequences. In a preferred embodiment, species of *Bacillus Licheniformis* make up at least 80%, at least 85%, at least 90%, at least 95% or at least 99% of the number of bacteria in the composition. In another preferred embodiment, species of *Bacillus Licheniformis* make up 100% of the bacteria in the composition.

The aerobic bacteria, as described above or their mixtures, may be isolated from suitable sources, such as from soil, digestive or hot water springs, or may be obtained from international strain collections of microorganisms, for example from DSMZ, CNCM or ATCC. The bacteria may be propagated by incubation at between 55 °C and 60 °C in rich medium, such as R2 broth (R2B; comprising 0.5 g yeast extract, 0.5 g proteose peptone #3, 0.5 g casamino acids, 0.5 g dextrose, 0.5 g soluble starch, 0.3 g sodium pyruvate, 0.3 g dipotassium phosphate and 0.05 g magnesium sulphate per liter H2O), or on minimal medium, such as M9 ( M9 comprising 12.8 g, Na₂HPO₄.7H₂O, 3 g KH₂PO₄, 0.5 g NaCl, 1 g NH₄Cl and 1 g of 1mM MgSO₄, 1mM CaCl₂, 3 x 10⁻⁹ M (NH₄)6Mo₇O₂₄.4H₂O, 4 x 10⁻⁷MH₃BO₃, 3x10⁻⁸ M CoCl₂.6H₂O, 1 x10⁻⁸ M CuSO₄.5H₂O, 8 x 10⁻⁸M MnCl₂.4H₂O,1x 10⁻⁸M ZnSO₄.7H₂O, 1 x10⁻⁶M FeSO₄.7H₂O). The bacterial formulation may further comprise vitamins, for example one or more of biotin, folic acid, nicotinic acid, riboflavin and thiamine; and trace elements, for example 0.01 g/l to 1 g/l of one or more of EDTA, MgSO₄ . 7H₂O, MnSO₄ . H₂O, FeSO₄ . 7H₂O, Co(NO₃)₂ . 6H₂O, CaCl₂ (anhydrous), ZnSO₄ . 7H₂O, CuSO₄ . 5H₂O, AlK(SO₄)₂ (anhydrous), H₃BO₃, Na₂MoO₄ . 2H₂O, Na₂SeO₃ (anhydrous), Na₂WO₄ . 2H₂O, NiCl₂ . 6H₂O.

After reaching the required yield, cells are harvested by centrifugation, dried, preferably lyophilized, and formulated with nutrients, vitamins, minerals and/or vegetable support for the bacteria, such as a nitrogen source, a phosphorus source, amino acids, zeolite, carbonate or wheat bran. In one embodiment, the bacteria are grown on an algal scaffold , for example Lithothamnium calcareum, which provides both nutritious elements, such as calcium, magnesium and iron, and volume to the formulation.

The formulation is preferably in dry form, for example a powder. The bacteria in the powder are in rest and become active when they come into contact with moisture. Since the formulation contains nutrients, vitamins and minerals to support bacterial growth, bacterial growth can start without much delay. The formulation preferably contains approximately 104 to 109 bacteria/g of the formulation. In one embodiment, 109 bacteria/g formulation is used. To determine cell concentration, cells are counted, for example by conventional techniques for agar plate cell counting.

The formulation may be comprised in a starter compost. The compost may be the product produced by the process of the present invention. In one embodiment, the starter compost comprises between 0.5% w/w (wt%) and 1% w/w (wt%) of the bacterial formulation.

The aerobic bacteria may be added to the digesting tank at the start of the batch operation and/or during the batch process. Addition during the batch process may be performed by an automated feeder which adds the aerobic bacteria at predetermined times and predetermined quantities during the batch operation. The bacteria are preferably present in a composition further comprising a bacterial growth medium being at least one member of the group consisting of maltodextrin, AGAR broth medium, *Lithothamnium Calcareum,* DNA and RNA nucleic acids, extracts of Laminariae Stipites, and extracts of Fucus Vesiculosus.

The digestion starts when the organic waste material is mixed with a composition comprising the above described bacteria at the claimed temperature preferably under moist conditions. The organic waste material is added to the digesting tank at the start of the batch operation. Preferably between 10 and 40 wt% of the content of the batch operation is a product obtained in a previous batch operation. By not emptying the entire content at the end of the batch operation this part may remain in the digesting tank. In this manner a starting quantity of the aerobic bacteria are present at the start of each batch operation. Preferably more than 70 wt% of the fresh organic waste material added to the digesting tank during a single batch operation is added at the start of the batch operation. Any remaining organic waste material is suitably added within 5 hours after starting the batch operation. At the end of a batch operation the content of the digesting tank is partly removed from the digesting tank and part of the wet digested material and bacteria remain in the apparatus for performing a next batch as described above.

In the process the moisture content of the organic waste material is preferably maintained at 60-80 wt.%. A faster decomposition of the organic waste material is possible according to this invention when the moisture content in the organic waste material is between 60 to 70 wt.%. When the organic waste material contains high levels of water it may thus be advantageous, energy-wise, to first lower this water content before performing the process. Removal of water may be performed by dewatering technologies known for manure processing and include a towerpress, a screwpress, a gravity belt thickener, a centrifuge or a decanter centrifuge. If the organic waste material comprises large pieces it may be preferred to reduce the size of these pieces before performing such a dewatering process.

When the organic waste material contains less water, for example less than 60 wt.%, as may be situation when the organic waste material comprises a substantial content of biodegradable plastics it may even be preferred to add water to the process. The added water may be tap water, waste water, rain water, preferably waste water.

The presence of water will result in a certain humidity of the air as present in the digesting tank and consequently of the humid air as discharged from the digesting tank. This humidity and temperature are favourable for the aerobic bacteria to perform. At the start of the digesting process humidity may be higher, for example at least 75% RH (relative humidity). At the end of the process, humidity may be as low as 14% RH. In one embodiment, humidity and temperature are time-controlled. For example, humidity is set for the first 3-8 hours at least 75% RH, the next 3-8 hours at between 60-70% RH, and, in the last 2- 8 hours , at 10-14% RH.

The temperature and humidity are preferably controlled. Temperature sensors and/or humidity sensors are preferably present to measure the temperature and humidity in the digesting tank. Humidity may be controlled by adapting the amount of air wherein more air will lower the humidity and less air will result in an increase in humidity.

When the organic waste comprises animal by-products, such as meat and/or fish waste, the following process has been found effective. The organic waste comprising meat and/or fish waste, especially category 3 waste, is intermittently supplied to the digesting tank over a batch period of 3 to 7 days while performing the process and wherein after adding the organic waste material the process is continued for a final period of between 10 and 50 hours. In this final period suitably no organic waste comprising such animal by-products such as meat and/or fish waste and especially category 3 waste, is supplied.

In the 3 to 7 days batch period a volume of organic waste material is bacterially decomposed to a digested material having a lower volume. The volume of intermittently supplied organic waste material is such that the combined volume of organic waste material and digested material does not exceed a predetermined upper value. Above such an upper value the digesting tank would contain a too high volume of solids which is not advantageous for a good mixing of the air and the waste material.

Preferably at the end of the final period between 60 and 90 wt% of the content of the digesting tank is discharged from the digesting tank resulting in a digesting tank containing digested material which digesting tank is used in a next batch period. This sequence allows for example a weekly batch process wherein in the first 5 days organic waste comprising animal by-products category 3 waste is supplied and in the final two days the organic waste is allowed to fully decompose to digested material.

To the digesting tank between 0.5 and 10 m³ of air per hour per kg of the total of organic waste material and digested material as present in the digesting tank is supplied. Suitably the supply of air is continuously and the discharge of air is continuously. The amount of air may vary, for example to control the humidity as explained above. The air is supplied while mixing the organic waste material and the aerobic bacteria. In this manner the oxygen as present in the air is effectively presented to the aerobic bacteria such that they can convert the organic waste material and the optional biodegradable plastic. Another function of this relatively large air flow is that the water as formed in the process is effectively removed from the reactor in the humid air stream. Because of the high volumes of air it is possible to directly discharge the humid air stream to the environment without having to take special measures for handling this water.

The air is preferably supplied using a high capacity ventilator. The air may be ambient air. The temperature of the air may be that of the ambient surroundings of the digesting tank. Preferably the temperature of the air as supplied is between 10 and 70 °C, preferably between 20° and 50° C.

The aerobic bacteria show optimal growth and activity at a temperature in the range of 40 to 70 °C. In the process of the present invention, the temperature of the organic waste material in the digesting tank is preferably maintained in the range of 40 to 65 or 40 to 50 °C, most preferably the temperature of the organic waste material is maintained at about 45 to 47 °C.

When the digested material is to be used as compost it is preferred that at the end of the batch operation the temperature is raised to above 65 °C and preferably between 65 and 72 °C, most preferably at 70 °C for a period of between 30 and 90 minutes. At these temperatures all or a large portion of pathogens, if present, will be effectively killed while the above mentioned aerobic bacteria such as the genus Geobacillus and Bacillus Licheniformis can resist these temperatures for this time period. In this manner most of the pathogens may be removed while obtaining a compost comprising the aerobic bacteria for use in a next batch as explained above.

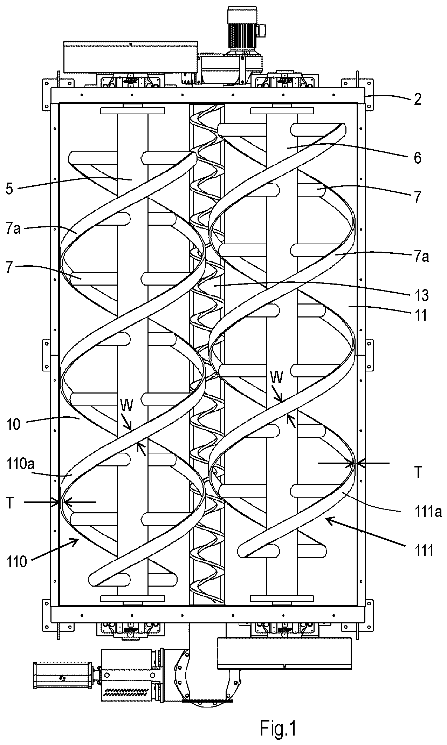

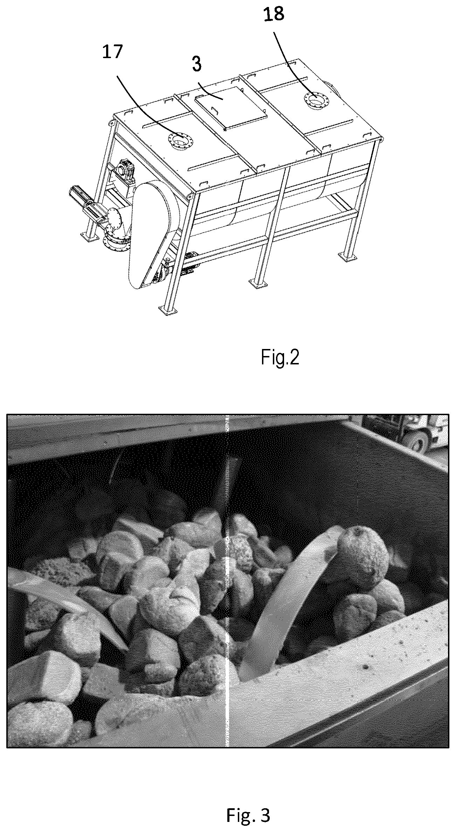

The digesting tank may be any tank known for such use. Examples are described in NL2018141. A preferred digesting tank is described in EP3581551. Reference is made to Figures 1 and 2. Preferably the digesting tank comprises an inlet (3) for the organic waste material, an outlet (4) for digested material and wherein the tank is provided with two rotating mixing shafts (5,6) provided with a helical mixing element connected to the shafts by supports (7) which radially extend from the shaft (5,6). The two rotating mixing shafts (5,6) are positioned substantially parallel with respect to each other in the elongated direction of the tank thereby defining two cylindrical mixing zones (8,9) in the tank. Below each of the two cylindrical mixing zones (8,9) a heated elongated semi-tubular surface (10,11) is provided as the lower inner wall of the tank. The temperature in the digesting tank is maintained by heat exchange between the heated elongated semi-tubular surfaces and the organic waste material present in the digesting tank. The volume of such a preferred digesting tank is between 3 and 20 m³ and more preferably between 5 and 17 m³.

The mixing shafts are designed to slowly mix the solids such that on the one hand oxygen from the air contacts the solids and on the other hand the solids remain sufficiently wet as described earlier for the bacteria to function. Preferably the mixing shafts rotate at a speed of between 0.8 and 1.2 rotations per minute. Between a rotation of a shaft a pause may be present of between 1 and 3 minutes. The two shafts may rotate simultaneously (in sync) of may rotate out of sync. The two shafts may also rotate in different directions. For example one shaft may perform a rotation while the other shaft does not rotate, when the first shaft is about half way its rotation the other shaft may start its rotation. After a pause the first shaft may perform a rotation in an opposite direction as compared to its first rotation while the other shaft performs a rotation in the same direction as its first rotation. In this manner the content of the digesting tank are well mixed and mixed with the air to perform the process. In this way enough oxygen will be present in the digesting tank to enhance the aerobic digestion.

The helical mixing element is suitably a blade (110,111) which runs at a certain distance from the semi-tubular surfaces (10,11). The blade (110,111) is suitably shaped such that the entire radially-outward facing surface (110a,111a) of the blade is spaced apart from the two semi-tubular surfaces (10,11) at a constant distance. It has been found that this distance is preferably minimal such that the blades may remove any deposits formed on the semi-tubular surfaces (10,11). Preferably this distance is smaller than 1 cm and more preferably smaller than 0.5 cm. The minimal distance will be determined by the requirement that the blades are spaced apart from the semi-tubular surfaces such that the mixing element may rotate within the tank (2). The radially-outwardly facing surfaces (110a, 111a) of the blades (110, 111) define a major surface area having a width (W) transverse to the blade's helical direction. The blades (110, 111) further define a minor surface area having a thickness (T) in the radial dimension. The width (W) is substantially greater than the thickness (T). The width (W) of the blades is preferably between 10 and 15 cm.

The distance between the radially outward facing surface and the semi-tubular surfaces is preferably less than 5 mm. This small distance avoids a build up of material on the semi-tubular surfaces and thus avoids that the heat transfer is negatively influenced.

Figure 2 shows the tank (2) of Figure 1 with a cover (16) and an inlet (3) for organic waste and an inlet (17) and outlet (18) for of air. As shown the air is supplied to the digesting tank by force at a position above the two cylindrical mixing zones and the humid air is discharged from the digesting tank at a position above the two cylindrical mixing zones.

In the process of this invention organic waste material optionally in combination with plastic disposables may be digested in a very short period of time, within 30 hours. This is much faster than state of the art digesting which typically takes several days to months. The digesting process is completed when an amorphous humus-like material is obtained, indicative that the waste has been converted to organic matter. Preferably, at least 80% w/w, at least 90% w/w, more preferably at least 95% w/w, at least 98% w/w or at least 99% w/w of the organic waste material has been converted into organic matter within 30 hours.

During digestion, the optional plastic in the organic waste material disappears, since it is also converted to organic matter. In one embodiment, at least 80% w/w of the plastic in the waste has disappeared within 30 hours. Most preferably, there are no traces of plastic visible after the digesting process according to the invention.

The invention shall be illustrated making use of the following examples.

### Example 1

A digesting tank as show in Figure 3a of EP3581551 having a distance between the helical mixing element and the semi-tubular surfaces of a few millimetres is used in this example. To this reactor 38 m³ of waste bread (15.200 kg was added. 100 kg digested material was already present in the reactor from a previous batch. This 100 kg previously digested material consisted also of the aerobic bacteria. 20 kg of fresh bacteria on a Lithothamnium Calcareum support was added after 3 hours and another. 5 kg of fresh bacteria was added after 6 hours. The mass in the reactor was stirred at a temperature of 45 °C and at an average rotation of 1 r.p.m. To the reactor 10.000 m³ air per hour was supplied using a ventilator and humid air was continuously discharged from the digester. The relative humidity of the humid air as discharged was over 70 %. After 7 hours the temperature was raised to 70 °C for one hour to kill any pathogens. Thereafter 70 wt% of the content of the digesting tank was discharged using the screw feeder.

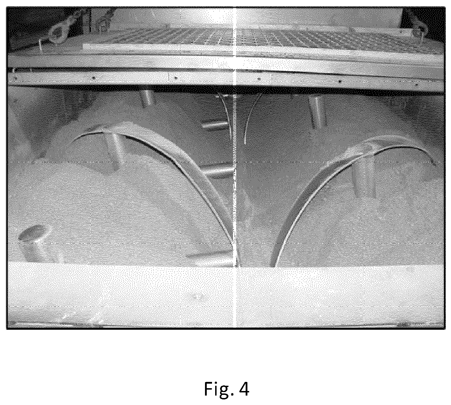

In Figure 3 a picture is shown of the starting situation and in Figure 4 a picture is shown just before discharging the digested material from the digesting tank. The digested material was analysed and found to be suited as an ingredient for sourdough bread replacing part of the flour and suited as livestock feed component.

### Example 2

Daily 4000 kg of organic waste material obtained from a fast food restaurant and comprising animal by-products category 3 waste, such as hamburgers, was physically dewatered to obtain 2400 kg of a dewater organic waste material. During a period of 5 days the daily amount of 2400 kg dewatered organic waste material was supplied daily to a digesting tank as show in Figure 3a of EP3581551. In the tank some 100 kg of digested material of a previous batch was present. 10.000 m³ air per hour was supplied to the digesting tank using a ventilator. The mass in the reactor was stirred at a temperature of 45 °C and at average rotation of 1 r.p.m at a relative humidity of the air of 70%. After one rotation a 2 minute pause was applied. The remaining organic waste is bacterially decomposed to about 810 kg of a digested material in two days. This reduction of mass and volume allows to add fresh dewatered organic waste material daily wherein the maximum mass of organic waste material and digested material did not exceed 6500 kg. After the 5 day batch period the process was continued under the same conditions and air flow without adding any fresh dewatered organic waste material.

The digested material had the properties as listed in table 1.

**Table1**

| | value |
|---|---|
| Dry solids (wt.%) | 94.7 |
| Volatiles at 550 °C (wt.%) | 74 |
| Potential to methane in an anaerobic digester (litre CH4/kg digested material) | 374 |

## Claims

1. Process to bacterially decompose organic waste material to a digested material in a digesting tank by mixing the organic waste material with aerobic bacteria at a temperature of between 40 C and 70 °C while supplying between 0.5 and 10 m³ of air per hour per kg of the total of organic waste material and digested material and discharging from the digesting tank a humid air stream having at least 60% relative humidity and wherein the organic waste further comprises one or more of the following biodegradable plastics selected from polybutyrate adipate terephthalate (PBAT), polylactic acid (PLA) and/or polyhydroxyalkanates (PHA) and wherein the biodegradable plastic content is in the range of between 1 and 30 wt% based on 100% dry matter of the organic waste material.

2. Process according to claim 1, wherein after performing the one step semi-batch process the temperature is raised in the digesting tank to between 65 and 72 °C for 30 to 90 minutes to kill pathogens followed by discharging all or part of the digested material from the digesting tank.

3. Process according to any one of claims 1-2, wherein the organic waste material comprises cellulosic, plant and/or animal waste.

4. Process according to claim 3, wherein the cellulosic, plant and/or animal waste is one or more of food substances, biological substances, vegetable waste, meat waste, fish waste and/or cellulose rich materials and wherein the organic waste comprises meat waste and/or fish waste is intermittently supplied to the digesting tank over a batch period of 3 to 7 days while performing the process and wherein after adding the organic waste material the process is continued for a final period of between 10 and 50 hours without adding meat waste and/or fish waste in this final period.

5. Process according to any one of claims 1-2, wherein the organic waste material is bread and/or dough and wherein the bacterially decomposing of the bread and/or dough is performed in between 8 and 24 hours.

6. Process according to any one of claims 1 to 5, wherein the aerobic bacteria belong to the genus *Geobacillus.*

7. Process according to any one of claims 1 to 6, wherein the aerobic bacteria belongs to the Bacillus Licheniformis.

8. Process according to claim 7, wherein the *Bacillus Licheniformis* is a *Bacillus Licheniformis* strain as deposited under the Budapest Treaty on April 1, 2021 at the Collection Nationale de Cultures de Microorganismes, and having accession number CNCM I-5567 or bacteria derived therefrom.

9. Process according to any one of claims 1-8, wherein the digesting tank comprises an inlet for the organic waste material, an outlet for digested material and wherein the tank is provided with two rotating mixing shafts provided with a helical mixing element connected to the shafts by supports which radially extend from the shaft, wherein the two rotating mixing shafts are positioned substantially parallel with respect to each other in the elongated direction of the tank thereby defining two cylindrical mixing zones in the tank,
wherein below each of the two cylindrical mixing zones a heated elongated semi-tubular surface is provided as the lower inner wall of the tank, and wherein the temperature in the digesting tank is maintained by heat exchange between the heated elongated semi-tubular surfaces and the organic waste material present in the digesting tank and wherein the air is supplied to the digesting tank by force at a position above the two cylindrical mixing zones and the humid air is discharged from the digesting tank at a position above the two cylindrical mixing zones.

10. Process according to claim 9, wherein the helical mixing elements are helical mixing blades and wherein each of the helical mixing blades comprises a radially-outwardly facing surface spaced apart from and facing the semi-tubular surfaces, the radially-outwardly facing surfaces each defining a major surface area having a width (W) transverse to the helical direction of between 10 and 15 cm, and wherein each of the helical mixing blades further comprises a minor surface area having a thickness in the radial dimension, and wherein the distance between the radially outward facing surface and the semi-tubular surfaces is less than 5 mm.

11. Use of the digested material as obtained from organic waste material comprising meat waste and/or fish waste according to the process of any one of claims 1-10 as a feedstock for an anaerobic digestion to biogas comprising methane and carbon dioxide.

12. Use of the digested material as obtained by the process according to any one of claims 5-10, wherein the organic waste material is bread and/or dough as part of a livestock feed.

13. Process to prepare a biogas comprising methane and carbon dioxide from organic waste material by performing a process to bacterially decompose an organic waste material with aerobic bacteria to a digested material according to a process according to any one of claims 1-10, at more than one location, transporting the digested material to a central location and performing at the central location an anaerobic digestion process using the digested material as feedstock to prepare biogas comprising methane and carbon dioxide.

14. Process according to claim 13, wherein more than 350 litre methane is produced per kg of digested material at the central location by the anaerobic digestion process.

15. Process to prepare a livestock feed composition by performing a process to bacterially decompose bread and/or dough with aerobic bacteria to a digested material according to a process according to any one of claims 5-10, at more than one location, transporting the digestive material to a central location and combining the digestive material with other livestock feed ingredients to prepare the livestock feed composition.

## Patentansprüche

1. Verfahren zum bakteriellen Zersetzen von organischem Abfall zu einem Faulgut in einem Faulbehälter durch Mischen des organischen Abfalls mit aeroben Bakterien bei einer Temperatur zwischen 40 °C und 70 °C unter Zufuhr von zwischen 0,5 und 10 m³ Luft pro Stunde pro kg der Summe aus organischem Abfall und Faulgut und Ablassen eines feuchten Luftstroms mit mindestens 60 % relativer Luftfeuchtigkeit aus dem Faulbehälter und wobei der organische Abfall ferner einen oder mehrere der folgenden biologisch abbaubaren Kunststoffe umfasst, ausgewählt aus Polybutyrat-Adipat-Terephthalat (PBAT), Polymilchsäure (PLA) und/oder Polyhydroxyalkanaten (PHA), und wobei der Gehalt an biologisch abbaubarem Kunststoff im Bereich zwischen 1 und 30 Gew.-%, bezogen auf 100 % Trockenmasse des organischen Abfalls, liegt.

2. Verfahren nach Anspruch 1, wobei nach dem Durchführen des einstufigen halbkontinuierlichen Verfahrens die Temperatur in dem Faulbehälter für 30 bis 90 Minuten auf auf zwischen 65 und 72 °C erhöht wird, um Krankheitserreger abzutöten, gefolgt von einem Ablassen des gesamten oder eines Teils des Faulguts aus dem Faulbehälter.

3. Verfahren nach einem der Ansprüche 1-2, wobei der organische Abfall cellulosehaltige, pflanzliche und/oder tierische Abfälle umfasst.

4. Verfahren nach Anspruch 3, wobei die cellulosehaltigen, pflanzlichen und/oder tierischen Abfälle eines oder mehrere von Lebensmittelsubstanzen, biologischen Substanzen, Gemüseabfällen, Fleischabfällen, Fischabfällen und/oder cellulosereichen Materialien ist und wobei der organische Abfall, der Fleischabfälle und/oder Fischabfälle umfasst, während der Durchführung des Verfahrens über einen Chargenzeitraum von 3 bis 7 Tagen intermittierend dem Faulbehälter zugeführt wird und wobei nach Zusetzen des organischen Abfalls das Verfahren für einen letzten Zeitraum zwischen 10 und 50 Stunden fortgesetzt wird, ohne in diesem letzten Zeitraum Fleischabfälle und/oder Fischabfälle zuzusetzen.

5. Verfahren nach einem der Ansprüche 1-2, wobei der organische Abfall Brot und/oder Teig ist und wobei die bakterielle Zersetzung des Brotes und/oder Teigs zwischen 8 und 24 Stunden durchgeführt wird.

6. Verfahren nach einem der Ansprüche 1 bis 5, wobei die aeroben Bakterien zur Gattung *Geobacillus* gehören.

7. Verfahren nach einem der Ansprüche 1 bis 6, wobei die aeroben Bakterien zu dem Bacillus Licheniformis gehören.

8. Verfahren nach Anspruch 7, wobei der *Bacillus Licheniformis* ein *Bacillus-Licheniformis-*Stamm, der gemäß dem Budapester Vertrag am 1. April 2021 bei der Collection Nationale de Cultures de Microorganismes hinterlegt wurde und die Zugangsnummer CNCM 1-5567 aufweist, oder davon abgeleitete Bakterien ist.

9. Verfahren nach einem der Ansprüche 1-8, wobei der Faulbehälter einen Einlass für den organischen Abfall und einen Auslass für Faulgut umfasst und wobei der Behälter mit zwei rotierenden Mischwellen bereitgestellt ist, die mit einem schraubenförmigen Mischelement bereitgestellt sind, das mit den Wellen durch Halterungen verbunden ist, die sich radial von der Welle erstrecken, wobei die beiden rotierenden Mischwellen im Wesentlichen parallel zueinander in der länglichen Richtung des Behälters positioniert sind, wodurch sie in dem Behälter zwei zylindrische Mischzonen definieren,
wobei unterhalb jeder der beiden zylindrischen Mischzonen eine beheizte längliche halbrohrförmige Fläche als untere Innenwand des Behälters bereitgestellt ist und wobei die Temperatur in dem Faulbehälter durch Wärmeaustausch zwischen den beheizten länglichen halbrohrförmigen Flächen und dem in dem Faulbehälter vorhandenen organischen Abfall aufrechterhalten wird und wobei die Luft dem Faulbehälter an einer Position oberhalb der beiden zylindrischen Mischzonen durch Kraft zugeführt wird und die feuchte Luft an einer Position oberhalb der beiden zylindrischen Mischzonen aus dem Faulbehälter abgelassen wird.

10. Verfahren nach Anspruch 9, wobei die schraubenförmigen Mischelemente schraubenförmige Mischschaufeln sind und wobei jede der schraubenförmigen Mischschaufeln eine radial nach außen weisende Fläche aufweist, die von den halbrohrförmigen Flächen beabstandet und ihnen zugewandt ist, wobei die radial nach außen weisenden Flächen jeweils einen größeren Flächenbereich mit einer Breite (W) quer zu der Schraubenrichtung zwischen 10 und 15 cm definieren und wobei jede der schraubenförmigen Mischschaufeln ferner einen kleineren Flächenbereich mit einer Dicke in der radialen Abmessung aufweist und wobei der Abstand zwischen der radial nach außen weisenden Fläche und den halbrohrförmigen Flächen weniger als 5 mm beträgt.

11. Verwendung des Faulguts, wie es aus organischem Abfall erhalten wird, umfassend Fleischabfälle und/oder Fischabfälle gemäß dem Verfahren nach einem der Ansprüche 1-10 als Ausgangsstoff für eine anaerobe Faulung zu Biogas, umfassend Methan und Kohlendioxid.

12. Verwendung des Faulguts, wie es durch das Verfahren nach einem der Ansprüche 5-10 erhalten wird, wobei der organische Abfall Brot und/oder Teig als Teil eines Viehfutters ist.

13. Verfahren zum Herstellen eines Biogases umfassend Methan und Kohlendioxid aus organischem Abfall durch Durchführen eines Verfahrens zum bakteriellen Zersetzen eines organischen Abfalls mit aeroben Bakterien zu einem Faulgut gemäß einem Verfahren nach einem der Ansprüche 1-10 an mehr als einem Ort, Transportieren des Faulguts zu einem zentralen Ort und Durchführen eines anaeroben Faulungsverfahrens an dem zentralen Ort unter Verwendung des Faulguts als Ausgangsstoff zum Herstellen von Biogas, das Methan und Kohlendioxid umfasst.

14. Verfahren nach Anspruch 13, wobei an dem zentralen Ort durch das anaerobe Faulverfahren mehr als 350 Liter Methan pro kg Faulgut erzeugt werden.

15. Verfahren zum Herstellen einer Viehfutterzusammensetzung durch Durchführen eines Verfahrens zum bakteriellen Zersetzen von Brot und/oder Teig mit aeroben Bakterien zu einem Faulgut gemäß einem Verfahren nach einem der Ansprüche 5-10 an mehr als einem Ort, Transportieren des Faulguts an einen zentralen Ort und Kombinieren des Faulguts mit anderen Viehfutterbestandteilen, um die Viehfutterzusammensetzung herzustellen.

## Revendications

1. Procédé de décomposition bactérienne de matière de déchets organiques en une matière digérée dans un réservoir de digestion en mélangeant de la matière de déchets organiques avec des bactéries aérobies à une température comprise entre 40 °C et 70 °C tout en alimentant de 0,5 à 10 m³ d'air par heure par kg de matière de déchets organiques et de matière digérée totale et en évacuant du réservoir de digestion un flux d'air humide ayant au moins 60 % d'humidité relative et dans lequel les déchets organiques comprennent en outre un ou plusieurs de ce qui suit : des plastiques biodégradables choisis parmi polybutylène adipate téréphtalate (PBAT), acide polylactique (PLA), et/ou polyhydroxyalcanoates (PHA) et dans lequel la teneur en plastique biodégradable est comprise dans la plage de 1 à 30 % en poids sur la base de 100 % de matière sèche de la matière de déchets organiques.

2. Procédé selon la revendication 1, dans lequel après la réalisation du procédé semi-continu en une étape, la température est élevée dans le réservoir de digestion à entre 65 et 72 °C pendant 30 à 90 minutes pour tuer des pathogènes, puis tout ou partie de la matière digérée est évacuée du réservoir de digestion.

3. Procédé selon l'une quelconque des revendications 1 et 2, dans lequel la matière de déchets organiques comprend des déchets cellulosiques, végétaux et/ou animaux.

4. Procédé selon la revendication 3, dans lequel les déchets cellulosiques, végétaux et/ou animaux sont l'un ou plusieurs de substances alimentaires, de substances biologiques, de déchets végétaux, de déchets de viande, de déchets de poisson et/ou de matières riches en cellulose et dans lequel les déchets organiques comprennent des déchets de viande et/ou des déchets de poisson alimentés de manière intermittente dans le réservoir de digestion pendant une période de lot de 3 à 7 jours pendant la réalisation du procédé et dans lequel après l'ajout de la matière de déchets organiques, le procédé est continué pendant une période finale comprise entre 10 et 50 heures sans ajout de déchets de viande et/ou de déchets de poisson pendant cette période finale.

5. Procédé selon l'une quelconque des revendications 1 et 2, dans lequel la matière de déchets organiques est du pain et/ou de la pâte et dans lequel la décomposition bactérienne du pain et/ou de la pâte est réalisée en 8 à 24 heures.

6. Procédé selon l'une quelconque des revendications 1 à 5, dans lequel les bactéries aérobies appartiennent au genre *Geobacillus.*

7. Procédé selon l'une quelconque des revendications 1 à 6, dans lequel les bactéries aérobies appartiennent à *Bacillus licheniformis.*

8. Procédé selon la revendication 7, dans lequel *Bacillus licheniformis* est une souche de *Bacillus licheniformis* telle que déposée selon le traité de Budapest le 1^{er} avril 2021 auprès de la Collection Nationale de Cultures de Microorganismes et ayant le numéro d'ordre CNCM 1-5567 ou des bactéries dérivées de celle-ci.

9. Procédé selon l'une quelconque des revendications 1 à 8, dans lequel le réservoir de digestion comprend une entrée pour la matière de déchets organiques, une sortie pour la matière digérée et dans lequel le réservoir est pourvu de deux arbres de mélange rotatifs pourvus d'un élément de mélange hélicoïdal relié aux arbres par des supports qui s'étendent radialement à partir de l'arbre, dans lequel les deux arbres de mélange rotatifs sont positionnés de manière sensiblement parallèlement l'un par rapport à l'autre dans la direction allongée du réservoir, définissant ainsi deux zones de mélange cylindriques dans le réservoir,
dans lequel en dessous de chacune des deux zones de mélange cylindriques, une surface semi-tubulaire allongée chauffée est fournie en tant que paroi interne inférieure du réservoir, et
dans lequel la température dans le réservoir de digestion est maintenue par un échange de chaleur entre les surfaces semi-tubulaires allongées chauffées et la matière de déchets organiques présente dans le réservoir de digestion et dans lequel l'air est fourni au réservoir de digestion par une force au niveau d'une position au-dessus des deux zones de mélange cylindriques et de l'air humide est évacué du réservoir de digestion au niveau d'une position au-dessus des deux zones de mélange cylindriques.

10. Procédé selon la revendication 9, dans lequel les éléments de mélange hélicoïdaux sont des lames de mélange hélicoïdales et dans lequel chacune des lames de mélange hélicoïdales comprend une surface orientée radialement vers l'extérieur espacée de et faisant face aux surfaces semi-tubulaires, les surfaces orientées radialement vers l'extérieur définissant chacune une étendue de surface majeure ayant une largeur (W) transversale à la direction hélicoïdale comprise entre 10 et 15 cm, et dans lequel chacune des lames de mélange hélicoïdales comprend en outre une étendue de surface mineure ayant une épaisseur dans la dimension radiale, et dans lequel la distance entre la surface orientée radialement vers l'extérieur et les surfaces semi-tubulaires est inférieure à 5 mm.

11. Utilisation de la matière digérée obtenue à partir de matière de déchets organiques comprenant des déchets de viande et/ou des déchets poisson selon le procédé selon l'une quelconque des revendications 1 à 10 comme matière première pour une digestion anaérobie en biogaz comprenant du méthane et du dioxyde de carbone.

12. Utilisation de la matière digérée obtenue par le procédé selon l'une quelconque des revendications 5 à 10, dans laquelle la matière de déchets organiques est du pain et/ou de la pâte comme partie d'aliment pour bétail.

13. Procédé pour préparer un biogaz comprenant du méthane et du dioxyde de carbone à partir de matière de déchets organiques en réalisant un procédé pour décomposer par voie bactérienne une matière de déchets organiques avec des bactéries aérobies en une matière digérée selon un procédé selon l'une quelconque des revendications 1 à 10, dans plus d'un emplacement, en transportant la matière digérée vers un emplacement central et en réalisant au niveau de l'emplacement central un procédé de digestion anaérobie à l'aide de la matière digérée comme matière première pour préparer du biogaz comprenant du méthane et du dioxyde de carbone.

14. Procédé selon la revendication 13, dans lequel plus de 350 litres de méthane sont produits par kg de matière digérée au niveau de l'emplacement central par le procédé de digestion anaérobie.

15. Procédé pour préparer une composition d'aliment pour bétail en réalisant un procédé pour décomposer par voie bactérienne du pain et/ou de la pâte avec des bactéries aérobies en une matière digérée selon un procédé selon l'une quelconque des revendications 5 à 10, dans plus d'un emplacement, en transportant la matière digérée vers un emplacement central et en combinant la matière digestive avec d'autres ingrédients d'aliment pour bétail pour préparer la composition d'aliment pour bétail.
